# EUROPEAN PATENT APPLICATION

(11) **EP 2 650 675 A1**
(43) Date of publication of application: **16.10.2013**
(21) Application number: 10860439.8
(22) Date of filing: 10.12.2010
(51) Int. Cl.: G01N 27/30, G01N 27/327, G01N 27/403, G01N 33/487, G01N 33/50, C12Q 1/00

(54) **METHOD FOR MANUFACTURING FLUID-DETECTING TEST PIECE**

(71) Applicant: Actherm Inc., Hsinchu 30078 (TW)
(72) Inventor: HSIEH, Wen-pin, Miaoli County 352 (TW); WANG, Cheng-hsien, Hsinchu City 300 (TW); WU, Ying-te, Tainan County 741 (TW); CHEN, Yi-chun, Hsinchu City 300 (TW)
(74) Representative: Lang, Christian
(86) International application number: PCT/CN2010/079669
(87) International publication number: WO 2012/075644

(57) **Abstract**

A method for manufacturing a fluid-detecting test piece is provided, which includes manufacturing a first and a second semi-finished goods. The manufacture of the first semi-finished goods includes: providing a substrate, forming multiple electrodes on the substrate, and forming a support layer which has multiple flow passage structures on the substrate with the formed electrodes directly. The manufacture of the second semi-finished goods includes: providing a lid, forming a hydrophilic layer on a first surface of the lid, and forming a glue layer on the rest of the hydrophilic layer of the first surface. Then the first and the second semi-finished goods are glued by matching the hydrophilic layer of the second semi-finished goods and the flow passage structures of the first semi-finished. The semi-finished assembly is scribed to form the fluid-detecting test piece.

## Description

### FIELD OF THE INVENTION

The present invention relates to a manufacturing method of a test strip, and more particularly, to a manufacturing method of a test strip for analyzing the biological material.

### BACKGROUND OF THE INVENTION

Test strips are conventionally implemented in biochemical detection and immunological detection. Fluid Channels or micro-channels are formed on the substrate or the base of the typical test strip. However, the fluid channel is surrounded by non-absorbent material, and the fluid samples to be tested are usually high viscous compositions, such as protein or carbohydrate. When the fluid sample flows in the fluid channel, some of the fluid sample will be left in the fluid channel, thus the fluid sample not completely reacted. Consequently, the fluid sample is wasted and errors may be occurred in the testing results.

In addition, the conventional test strips are provided with micro-channels for delivering the fluid. By implementing the capillarity of the micro-channel structure, the fluid sample is drawn to flow through the fluid channel to the reaction/detection region. Alternatively, the fluid sample is injected by an external pressure to drive the fluid sample flowing through the fluid channel to the reaction/detection region. However, in any of the methods described above, there are some air bubbles with various sizes generated after the fluid sample is introduced into the fluid channel. These bubbles, when causing the fluid channel blocked, may result in test errors or even test failures.

Besides, during batch manufacturing of the conventional test strip, the test strip is manufactured by stacking method as shown in US patent 6,258,229. At first, an electrode layer is printed on a large substrate, and the electrodes of the electrode layer are arranged repeatedly. The number of the electrodes in one pattern is variable in accordance with the practical requirement (such as: two electrodes type, three electrodes type, four electrodes type, or multiple electrodes type). Thereafter, the insulating layer with a plurality of long-shaped openings (such as the channel of the test strip) is adhered to the substrate printed with electrodes. Each of the long-shaped openings is aligned to the end of the electrodes and a reactive material is provided in each of the long-shaped opening. Then, the lid with a plurality of air vents is adhered to the insulating layer. Each of the air vents is located above the end of each long-shaped opening, thus completing the manufacture of the test strip assembly. Finally, the test strip assembly is cut into a plurality of test strips.

According to the above-mentioned manufacturing procedures, when the insulating layer is adhered to the substrate, the fluid channel of the insulating layer is required to do one dual-axis alignment with the electrode of the substrate before adhering. Also, before the lid is adhered to the insulating layer, the air vent of the lid is required to do another dual-axis alignment with the fluid channel.

### SUMMARY OF THE INVENTION

In order to overcome the abovementioned shortcomings, the present invention provides a manufacturing method of a test strip for a fluid sample, which comprises the following steps:
(1) making a first semi-finished product by steps of:
   (1-1) providing a first substrate with a first axis and a second axis, and the first axis and the second axis being orthogonal to each other;
   (1-2) forming a plurality of electrodes on the substrate with the electrodes being disposed in parallel with the second axis;
   (1-3) directly forming a supporting layer on the substrate having the electrodes with the supporting layer including a plurality of fluid channels disposed in array pattern along the first axis and correspondingly on the electrodes, and with a thickness of the supporting layer being at least 30 micrometers (µm); and
   (1-4) filling the fluid channels with a reactive material to form the first semi-finished product;
(2) making a second semi-finished product by steps of:
   (2-1) providing a lid having a first surface, a second surface, a third axis and a fourth axis, the first surface being opposite to the second surface and the third axis being orthogonal to the fourth axis;
   (2-2) forming a hydrophilic layer on the first surface of the lid; and
   (2-3) forming an adhering layer on the first surface of the lid without the hydrophilic layer to form the second semi-finished product;
(3) matching the hydrophilic layer of the second semi-finished product to the fluid channels of the first semi-finished product;
(4) adhering the first semi-finished product and the second semi-finished product to form a test strip assembly; and
(5) cutting the test strip assembly along the first axis to produce a plurality of test strips with each of the test strips having one fluid channel, wherein the fluid channel of each of the test strips, the lid, and the substrate defines a sensing region.

In the abovementioned manufacturing method, a volume of the sensing region is at least 0.3 µl.

In the abovementioned manufacturing method, the step of making the second semi-finished product further comprises a step of disposing a release layer on the adhering layer of the second semi-finished product.

In the abovementioned manufacturing method, the method further comprises a step of removing the release layer before the step of adhering the first semi-finished product and the second semi-finished product.

In the abovementioned manufacturing method, the step of directly forming the supporting layer on the substrate having the electrodes is coating an insulating material on the substrate having the electrodes and solidifying the insulating material by providing a light energy on the insulating material.

In the abovementioned manufacturing method, the step of directly forming the supporting layer on the substrate having the electrodes is coating an insulating material on the substrate having the electrodes and solidifying the insulating material by heating the insulating material.

In the abovementioned manufacturing method, the method further comprises a step of forming an ink structure on the first surface of the lid before the step of forming the adhering layer on the second semi-finished product.

In the abovementioned manufacturing method, the method of forming the electrodes on the substrate is one selected from the group consisting of printing, spreading and deposition.

In the abovementioned manufacturing method, the method of forming the electrodes on the substrate is one selected from the group consisting of electroplating, vapor depositing and sputtering.

In the abovementioned manufacturing method, the step of forming the hydrophilic layer on the first surface of the lid is performed by relief printing.

Hence, the advantageous effects provided by the manufacturing method of the test strip for the fluid sample in the present invention as follows:

Since the supporting layer is directly formed on the substrate and the alignment process is not required to execute, thus avoiding the operation mistakes occurred and the resultant damages in the substrate, the insulating layer or the semi-finished products, thereby reducing the manufacturing cost but improving the yield.

### DESCRIPTION OF THE DRAWINGS

FIG. 1 is a flow chart illustrating the manufacturing method of the test strip for the fluid sample according to the preferred embodiment of the present invention;
FIG. 2 is a schematic view illustrating the substrate in the preferred embodiment of the present invention;
FIG. 3 is a schematic view illustrating the manufacturing method of the electrodes in the preferred embodiment of the present invention;
FIG. 4 is a schematic view illustrating the manufacturing method of the supporting layer in the preferred embodiment of the present invention;
FIG. 5 is a schematic view illustrating the first semi-finished product in the preferred embodiment of the present invention;
FIG. 6 is a schematic view illustrating the second semi-finished product in the preferred embodiment of the present invention;
FIG. 7 is a schematic view illustrating an assembly of the first semi-finished product and the second semi-finished product in the preferred embodiment of the present invention;
FIG. 8 is a schematic view illustrating the test strip assembly in the preferred embodiment of the present invention;
FIG. 9 is a schematic view illustrating the cutting mode of the test strip assembly in the preferred embodiment of the present invention;
FIG. 10 is a schematic view illustrating the single test strip after the cutting process in the preferred embodiment of the present invention; and
FIG. 11 is a schematic sectional view illustrating the test strip along the BB line of FIG. 10 in the preferred embodiment of the present invention.

### DETAILED DESCRIPTION OF THE PREFERRED EMBODIMENTS

The present invention discloses a manufacturing method of a test strip for a fluid sample in which the detecting theory for the biological sample as well as the liquid coating technique are well known to those skilled in the art. Therefore, a detail description of such principles and techniques is omitted herein for brevity. Besides, the drawings referred to in the following description are not drawn to actual scale and need not be so because they are intended to demonstrate features of the present invention only schematically.

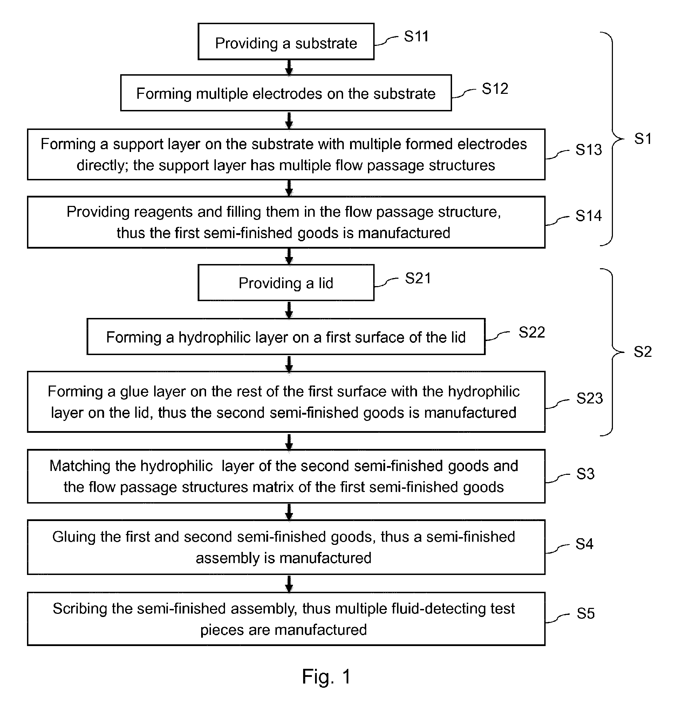

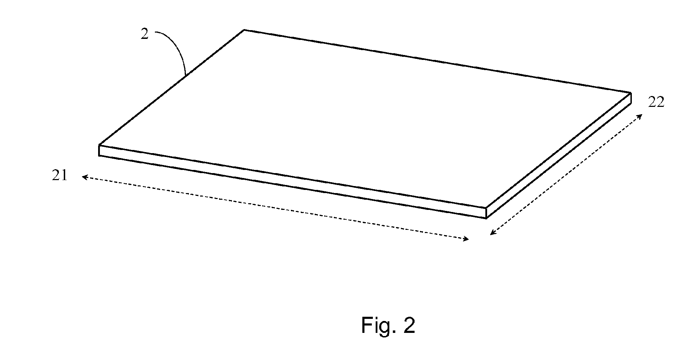

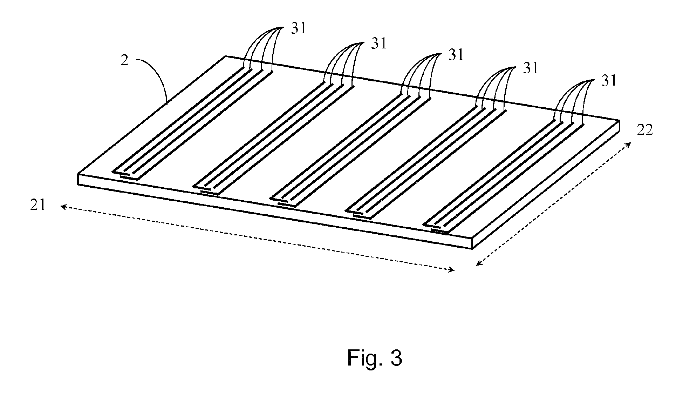

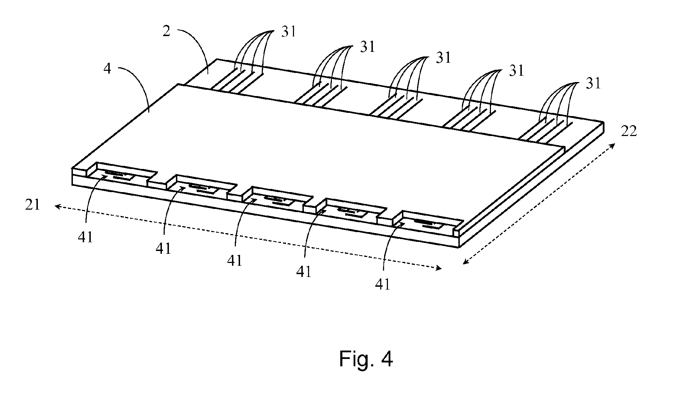

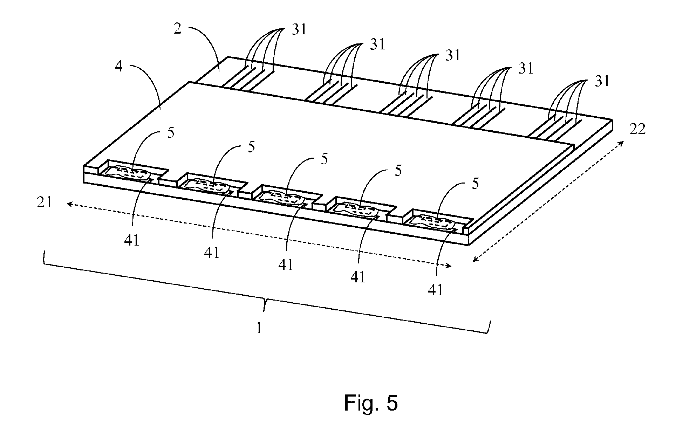

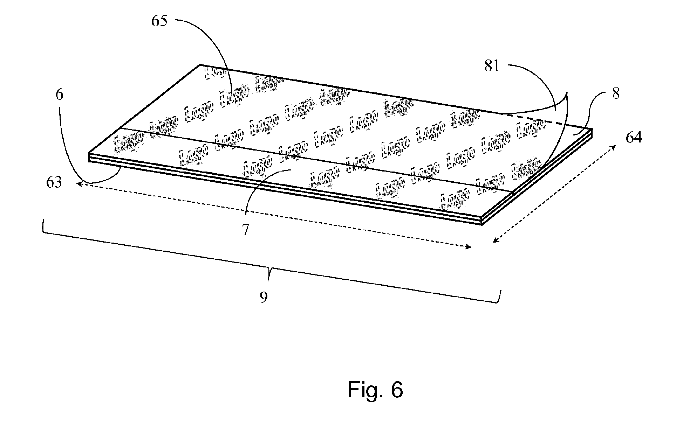

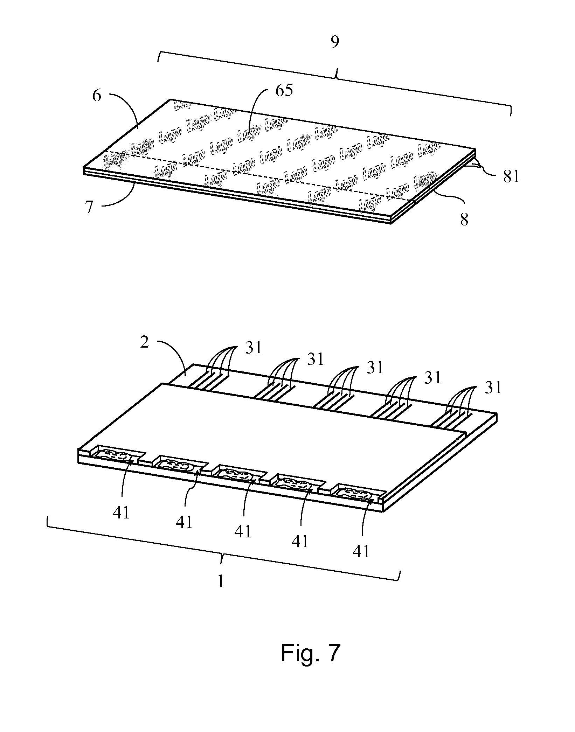

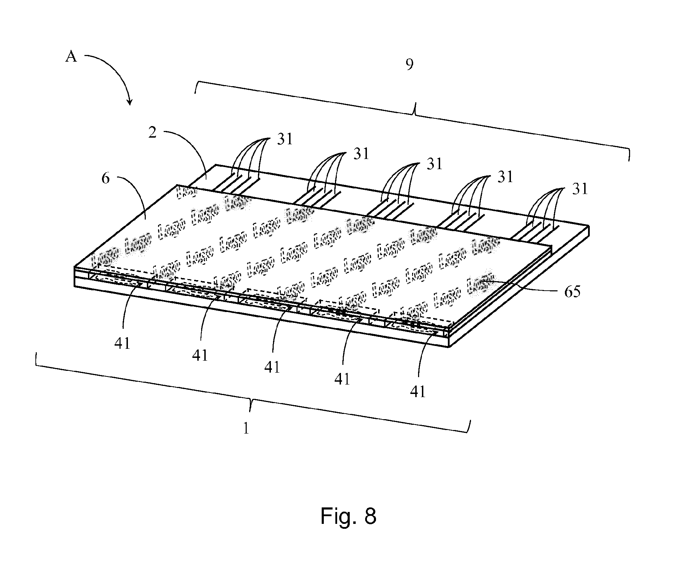

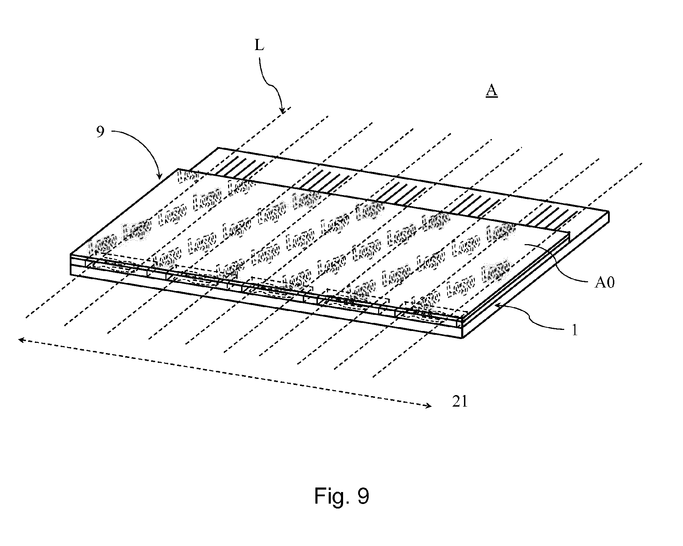

Firstly, please refer to FIG. 1, which is a flow chart illustrating the manufacturing method of the test strip for the fluid sample according to the preferred embodiment of the present invention and the test strip for the fluid sample comprises steps of:
Step S1: Step S1 includes step S11 to step S 14 and the purpose thereof is to make a first semi-finished product having a substrate, a plurality of electrodes and a supporting layer. Please refer to FIG. 2, which is a schematic view illustrating the substrate in the preferred embodiment of the present invention.
Step S11: First, the present invention provides a substrate 2 and the substrate 2 has a first axis 21 and a second axis 22. The first axis 21 and the second axis 22 are orthogonal to each other.
Step S12: Then, please refer to FIG. 3, which is a schematic view illustrating the manufacturing method of the electrodes in the preferred embodiment of the present invention. A plurality of electrodes 31 are formed on the substrate 2 and the forming method implemented herein is printing (such as screen printing or relief printing), spreading or deposition. Each of the electrodes is disposed in parallel with the second axis 22. In addition, the electrodes 31 are formed on the substrate 2 by a mask with electroplating, vapor planting or sputtering.
Step S13: Please refer to FIG. 4, which is a schematic view illustrating the manufacturing method of the supporting layer in the preferred embodiment of the present invention. After the electrodes 31 are formed on the substrate 2 by printing, the supporting layer 4 is directly formed on the electrodes 31 and includes a plurality of fluid channels 41. The fluid channels 41 are disposed in array pattern along the first axis 21 and correspondingly on the electrodes 31. In addition, the thickness of the supporting layer 4 is at least 30 micrometer (µm). Moreover, the fluid channels 41 described herein is not only provided for the fluid flowing through but also provided with an air vent for guiding the emission of the gas, thus preventing the occurrence of the obstructed event.
   During manufacturing the supporting layer 4, the thick film process such as printing (for example, mask printing), imprinting, spreading or deposition and so on is used as well as the insulating material is directly coated on the substrate 2 with the electrodes 31. Besides, the fluid channel 41 is disposed in array pattern during the coating process described above. The insulating material used herein can be optical sensitive material or thermosetting material. After coating or printing, the insulating material is directly cured by light or heat to solidify and form the supporting layer 4 on the electrode 31. At the same time, the fluid channel 41 with array pattern is solidified and formed thereon. For example, the UV glue is used to be the insulating material to form the supporting layer 4 on the substrate 2 with the electrodes 31 and then ultraviolet light is used to solidify the insulating material. Alternatively, the Epoxy resin is used to coat on the substrate 2 with the electrodes 31, and then an infrared light lamp or a heating plate is used to provide heat to solidify and form the supporting layer 4. The abovementioned method of using the infrared light lamp is implemented by the heat of the infrared light instead of the light of the infrared light wave band to heat and solidify the insulating material.
Step S 14: Next, please refer to FIG. 5, which is a schematic view illustrating the first semi-finished product in the preferred embodiment of the present invention. The reactive material 5 is used to fill the fluid channels 41 after manufacturing the supporting layer 4 by the thick film process. Therefore, the first semi-finished product is formed according to step S11 to step S 14.
Step S2: Step S2 includes step S21 to step S23, and the purpose thereof is to make a second semi-finished product having a lid with a hydrophilic layer and an adhering layer.
Step S21: Firstly, the lid 6 is provided and includes a first surface 61, a second surface 62, a third axis 62 and a fourth axis 64. The first surface 61 is opposite to the second surface 62 and the third axis 63 is orthogonal to the fourth axis 64.
Step S22: Next, the hydrophilic layer 7 is formed along the third axis 63 on the first surface 61 of the lid 6, and the method of forming the hydrophilic layer is preferably performed by relief printing.
Step S23: Thereafter, the adhering layer 8 is formed on the first surface 61 of the lid 6 without the hydrophilic layer 7. Therefore, the second semi-finished product is formed according to step S21 to step S23.
Please refer to FIG. 6, which is a schematic view illustrating the second semi-finished product manufactured by the abovementioned step S21 to step S23 in the preferred embodiment of the present invention. In addition, before the adhering layer 8 is formed, an ink structure 65 is formed on the first surface 61 of the lid 6 to be a pattern including a logo or any other pictures. Hence, as the lid 6 is transparent, the pattern of the ink structure formed on the downside of the lid 6 can be seen from the upside of the lid 6.
   In order to prevent the pollution of the adhering layer 8 or the adhesion occurred between the adhering layer 8 of each of the second semi-finished products 9 and the lid 6 or other areas of other second semi-finished products 9, a release layer is disposed on the adhering layer after forming the adhering layer to prevent from the occurrence of the abovementioned events.
Step 3: Please refer to FIG. 7, which is a schematic view illustrating the assembly of the first semi-finished product 1 and the second semi-finished product 9 in the preferred embodiment of the present invention. The hydrophilic layer 7 of the second semi-finished product 9 made by step 2 is matched to the fluid channels 41 of the first semi-finished product 1 made by step 1. Besides, the hydrophilic layer 7 of the second semi-finished product 9 is needed to align with the fluid channels 41 of the first semi-finished product 1.
Step 4: Next, please refer to FIG. 8, which is a schematic view illustrating the test strip assembly in the preferred embodiment of the present invention. After the hydrophilic layer 7 of the second semi-finished product 9 made by step 2 is matched to the fluid channels 41 of the first semi-finished product 1 made by step 1, the first semi-finished product 1 and the second semi-finished product 9 are adhered to each other by the adhering force provided by the adhering layer 8 (as shown in FIG. 5) to be the test strip assembly A. Further, if the release layer 81 (as shown in FIG. 5) is disposed on the adhering layer 8 (as shown in FIG. 5), the release layer 81 is removed before the adhering process implemented between these two semi-finished products.
Step 5: Please refer to FIG. 9, which is a schematic view illustrating the cutting mode of the test strip assembly in the preferred embodiment of the present invention. After the first semi-finished product 1 and the second semi-finished product 9 are adhered to each other to form the test strip assembly A, the test strip assembly A is cut along the first axis 21 as the cutting line L shown in dot line of the figure to produce a plurality of test strips A0 for the fluid sample.

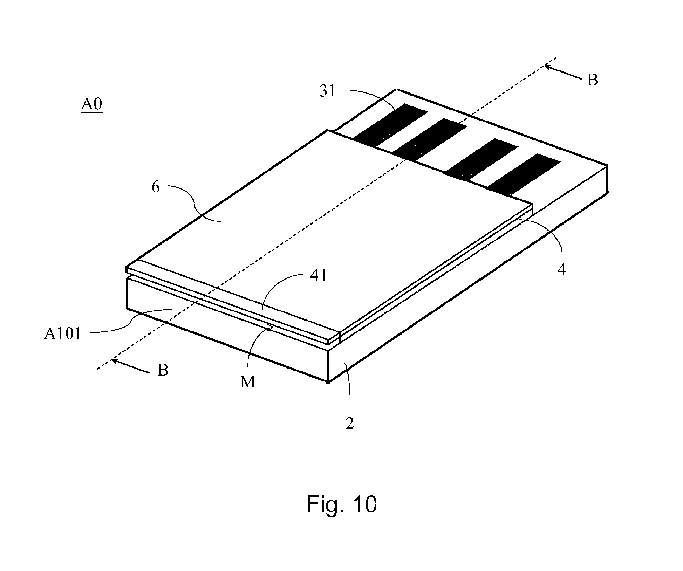

Please refer to FIG. 10, which is a schematic view illustrating the single test strip after the cutting process manufactured by the abovementioned step S1 to step S5 in the preferred embodiment of the present invention. The test strip A0 for the fluid sample includes a substrate 2, a plurality of the electrodes 31, a supporting layer 4 and a lid 6. The material of the substrate A0 is preferred to be the bio-inert material. Each of the test strip A0 includes one fluid channel 41 and the fluid channel 41, the lid 6 and the substrate 2 are together to define a sensing region M. The volume of the sensing region M is at least 0.3 µl and preferred to be 0.5∼1 µl. Moreover, the rationale of directly printing the supporting layer 4 on the electrodes 31 by thick coating in the present invention is to omit the accurate alignment procedure during adhering, thereby preventing from the manufacturing failure because of the aberration alignment. In addition, the thickness of the supporting layer 4 determines the size of the sensing region M and the procedure of thick coating can accurately provide a fixed reactive space for reacting.

The lid 6 is disposed on the supporting layer 4 as well as the sensing region M is disposed in the first end A101 of the test strip A0 and defined by the supporting layer 4, the lid 6 and the substrate 2. Besides, the lid 6 completely covers the sensing region M, and the sensing region M is disposed in parallel with the horizontal short axis of the test strip A0. By the disposition provided in the present invention, the user just needs to let the first end A101 of the test strip A0 to be close to the site of sampling (for example, the acupuncturing site of skin) during usage, then the fluid sample could be facilitated to enter the sensing region M by capillary action, thus making sampling more convenient.

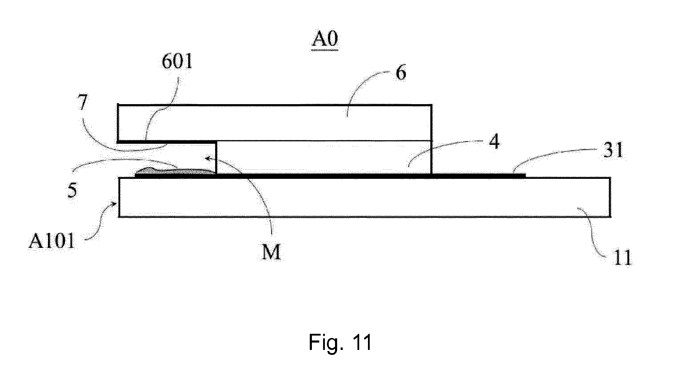

Please refer to FIG. 11, which is a schematic view illustrating the test strip A0 along the BB line of FIG. 8 in the preferred embodiment of the present invention. The lid 6 is disposed on the supporting layer 4 to completely cover the sensing region M. Therefore, a cantilever structure is formed on the first end A101 (as shown in FIG. 8) of the test strip A0 and the electrode 31 is extended to the area of the sensing region M. The sensing region M is defined by the supporting layer 4, the lid 6 and the substrate 2 and includes a C shaped structure as shown in figure. In addition, the hydrophilic layer 7 is coated on one side 601 of the lid 6 facing the sensing region M for facilitating the fluid sample smoothly flowing into the sensing region M. The sensing region M also includes the dried reactive material 5. Moreover, in order to observe the situation during the testing sample flowing into the sensing region M, the area close to the first end A101 of the lid 6 is preferred to be transparent material to avoid the error derived from insufficient testing sample being not enough to fill the sensing region M.,.

As described above, the present invention has been described with preferred embodiments thereof and it is understood that many changes and modifications to the described embodiments can be carried out without departing from the scope and the spirit of the invention that is intended to be limited only by the appended claims.

## Claims

1. A manufacturing method of a test strip for a fluid sample, comprising steps of:
making a first semi-finished product by steps of:
providing a substrate defined by a first axis and a second axis being orthogonal to each other;
forming a plurality of electrodes on the substrate with the electrodes being disposed in parallel with the second axis;
directly forming a supporting layer on the substrate having the electrodes with the supporting layer including a plurality of fluid channels disposed in array pattern along the first axis and correspondingly on the electrodes, and with a thickness of the supporting layer being at least 30 micrometers; and
filling the fluid channels with a reactive material;
making a second semi-finished product by steps of:
providing a lid having a first surface and a second surface and being defined by a third axis and a fourth axis with the first surface being opposite to the second surface and the third axis being orthogonal to the fourth axis;
forming a hydrophilic layer on the first surface of the lid; and
forming an adhering layer on the first surface of the lid without the hydrophilic layer;
matching the hydrophilic layer of the second semi-finished product to the fluid channels of the first semi-finished product;
adhering the first semi-finished product and the second semi-finished product to form a test strip assembly; and
cutting the test strip assembly along the first axis to produce a plurality of test strips with each of the test strips having one fluid channel, wherein the fluid channel of each of the test strips, the lid, and the substrate defines a sensing region.

2. The manufacturing method of the test strip for the fluid sample of claim 1, wherein a volume of the sensing region is at least 0.3 µl.

3. The manufacturing method of the test strip for the fluid sample of claim 1, wherein the step of making the second semi-finished product further comprises a step of disposing a release layer on the adhering layer of the second semi-finished product.

4. The manufacturing method of the test strip for the fluid sample of claim 3, further comprising a step of removing the release layer before the step of adhering the first semi-finished product and the second semi-finished product.

5. The manufacturing method of the test strip for the fluid sample of claim 1, wherein the step of directly forming the supporting layer on the substrate having the electrodes is coating an insulating material on the substrate having the electrodes and solidifying the insulating material by providing a light energy on the insulating material.

6. The manufacturing method of the test strip for the fluid sample of claim 1, wherein the step of directly forming the supporting layer on the substrate having the electrodes is coating an insulating material on the substrate having the electrodes and solidifying the insulating material by heating the insulating material..

7. The manufacturing method of the test strip for the fluid sample of claim 1, further comprising a step of forming an ink structure on the first surface of the lid before the step of forming the adhering layer on the first surface of the lid without the hydrophilic layer.

8. The manufacturing method of the test strip for the fluid sample of claim 1, wherein the method of forming the electrodes on the substrate is one selected from the group consisting of printing, spreading and deposition.

9. The manufacturing method of the test strip for the fluid sample of claim 1, wherein the method of forming the electrodes on the substrate is one selected from the group consisting of electroplating, vapor depositing and sputtering.

10. The manufacturing method of the test strip for the fluid sample of claim 1, wherein the step of forming the hydrophilic layer on the first surface of the lid is performed by relief printing.
